(19) Europäisches Patentamt
European Patent Office
Office européen des brevets

(11) EP 2 586 811 B1

(12) **EUROPEAN PATENT SPECIFICATION**

(45) Date of publication and mention of the grant of the patent:
**20.11.2019 Bulletin 2019/47**

(21) Application number: **11798136.5**

(22) Date of filing: **21.06.2011**

(51) Int Cl.:
***C08G 65/32*** *(2006.01)*
***A61K 47/60*** *(2017.01)*
***C08G 65/26*** *(2006.01)*
***C08G 65/325*** *(2006.01)*
***C08G 65/331*** *(2006.01)*
***C08G 65/334*** *(2006.01)*
***C08G 65/333*** *(2006.01)*

(86) International application number:
**PCT/JP2011/064154**

(87) International publication number:
**WO 2011/162252 (29.12.2011 Gazette 2011/52)**

(54) **BRANCHED HETERO-POLYETHYLENE GLYCOL AND INTERMEDIATE**

VERZWEIGTES HETERO-POLYETHYLENGLYCOL UND ZWISCHENPRODUKT DAVON

HÉTÉRO-POLYÉTHYLÈNE GLYCOL RAMIFIÉ ET INTERMÉDIAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.03.2011 JP 2011070735**
**25.06.2010 JP 2010145383**

(43) Date of publication of application:
**01.05.2013 Bulletin 2013/18**

(73) Proprietor: **NOF Corporation**
**Shibuya-ku**
**Tokyo 150-6019 (JP)**

(72) Inventors:
- **YAMAMOTO Yuji**
**Kanagawa 210-0865 (JP)**
- **YOSHIOKA Hiroki**
**Kanagawa 210-0865 (JP)**
- **MANABE Fumiaki**
**Kanagawa 210-0865 (JP)**

(74) Representative: **Hoffmann Eitle**
**Patent- und Rechtsanwälte PartmbB**
**Arabellastraße 30**
**81925 München (DE)**

(56) References cited:
**EP-A1- 1 857 462**
**WO-A1-02/060978**
**WO-A2-03/040211**
**JP-A- 5 279 469**
**JP-A- 7 048 449**
**JP-A- 7 048 450**
**JP-A- 11 228 685**
**JP-A- 11 228 685**
**JP-A- 2000 001 541**
**JP-A- 2000 001 541**
**JP-A- 2001 519 784**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 586 811 B1

## Description

Technical Field

The present invention relates to a branched hetero polyethylene glycol. More specifically, the invention relates to a branched hetero polyethylene glycol compound which is used for modification of bio-functional molecules, drugs or drug carriers in drug delivery systems, materials and devices for diagnosis, or the like.

Background Art

A polyethylene glycol itself exhibits low toxicity and antigenicity and has excellent solubility toward water and many organic solvents. Therefore, end-activated polyethylene glycol compounds where a reactive functional group is introduced into an end thereof have been widely used for imparting various functions, for example, imparting stealthiness of drugs and drug carriers in the body and solubilizing them in drug delivery systems, and improving bio-compatibility of material surfaces. Of the compounds, a so-called hetero bifunctional polyethylene glycol having functional groups different in reactivity at two ends of a linear polyethylene glycol, respectively, can introduce different molecules, for example, bio-functional molecules such as drugs, physiologically active substances, targeting substances, and the like into respective ends. Accordingly, the polyethylene glycol is used as a hetero crosslinker which crosslinks these bio-functional molecules each other or the bio-functional molecules and various drug carriers or devices.

A first characteristic advantage obtained by using the hetero bifunctional polyethylene glycol as such a hetero crosslinker is (A) a fact that low antigenicity and excellent solubility, which are properties of a polyethylene glycol as described above, can be imparted to the bonded substance. Since a polyethylene glycol is a polymer having repeating units, it has considerable molecular weight and number of monomer units and the distance between the ends is long as compared with a usual low-molecular-weight hetero crosslinker. Therefore, the next advantage is (B) a fact that substances which are difficult to bond directly owing to steric hindrance or molecules or substances which may lose a function intrinsic to the molecules, such as pharmacological activity, by direct bonding, or substances having different natures, such as molecules or substances and various drug carriers or devices can be bonded each other.

In the field of DDS, there are many studies on conjugates using the hetero bifunctional polyethylene glycol.

Jiang et al. (Non-Patent Document 1) have bonded transferrin to β-lactoglobulin using a hetero bifunctional polyethylene glycol having an N-hydroxysuccinimidoester group and a maleimide group. Zhang et al. (Non-Patent Document 2) have bonded NLS (nuclear localization signal) that is a peptide decamer to a dendrimer bonded substance of acridine using a hetero bifunctional polyethylene glycol having an N-hydroxysuccinimidoester group and a maleimide group. Kim et al. (Non-Patent Document 3) have bonded siRNA to LHRH (luteinizing hormone releasing hormon) using a hetero bifunctional polyethylene glycol having an amino group or a 2-pyridyldisulfide group and a carboxylic acid. Anhorn et al. (Non-Patent Document 4) have bonded an IgG antibody to a nanoparticle including an anticancer agent using a hetero bifunctional polyethylene glycol having an N-hydroxysuccinimidoester group and a maleimide group.

Moreover, as an application to diagnostic devices, Otsuka et al. (Non-Patent Document 5) have showed utilization as a biosensor which monitors a bio-functional molecule such as lectin, where lactose is bonded to polylactic acid using a hetero bifunctional polyethylene glycol having an acetal group and a hydroxyl group and, further utilizing interaction between lactose and lectin and interaction between polylactic acid and an inorganic substance surface, as a result, lectin and the inorganic substance surface is crosslinked.

As above, a hetero bifunctional polyethylene glycol is a hetero crosslinker widely used in medicament-related uses such as modification of bio-functional molecules, especially in DDS filed. On the other hand, when it is considered to enhance the function of the polyethylene glycol bonded substance where the hetero bifunctional polyethylene glycol is reacted with various bio-functional molecules, carriers, and the like, there is a case where plural modification of either one bio-functional molecule is desired. For example, in the case where a polyethylene glycol bonded substance having a drug at one end and a targeting molecule at another end bonded thereto is considered, when plural bonding of the drug is achieved, it becomes possible to improve transport efficiency. Moreover, when plural bonding of the targeting molecule is achieved, it becomes possible to improve targeting performance toward a ligand.

As a polyethylene glycol compound taking such plural modification of the bio-functional molecule into consideration, there is a description of so-called Forked-PEG in US6362254 (Patent Document 1).

In the Patent, though there is no application example to hetero polyethylene glycol derivatives, Forked-PEG where two functional groups are introduced into one polyethylene glycol end has been synthesized for creating a branching point at one end of a methoxy-polyethylene glycol and for modifying a bio-functional molecule. WO03040211 is directed to branched reactive water-soluble polymers comprising at least two polymer arms, such as poly(ethylene glycol), attached to a central aliphatic hydrocarbon core molecule through heteroatom linkages. The branched polymers bear at least one functional group for reacting with a biologically active agent to form a biologically active conjugate.

JPH11228685 and JP2000001541 demonstrate carboxyl or amino group-containing polyoxyalkylene compound useful for medical use.

Prior Art Documents

Patent Document

Patent Document 1: U. S. Patent No. 6362254

Non-Patent Documents

Non-Patent Document 1: Journal of Drug Targeting, 2007, 15(10), 672-683
Non-Patent Document 2: BioconjugateChem., 2009, 20, 120-128
Non-Patent Document 3: BioconjugateChem., 2008, 19, 2156-2162
Non-Patent Document 4: BioconjugateChem., 2008, 19, 2321-2331
Non-Patent Document 5: Langmuir, 2004, 20(26), 11285-11287

Disclosure of the Invention

Problems that the Invention is to Solve

However, in Patent Document 1, the length between the branching point and plurality of functional groups is not definitely defined and described examples are limited to those having a very near distance between two functional groups. When introduction of identical bio-functional molecules, especially substances having a very large molecular weight, such as proteins or antibodies, into the two functional groups are supposed, this means that an advantage that "(B) molecules which are difficult to bond directly owing to steric hindrance or molecules which may lose a function intrinsic to the molecules, such as pharmacological activity, by direct bonding can be bonded each other", which is a remarkable characteristic of the hetero polyethylene glycol derivative, is impaired to a large extent. Therefore, as an application to a hetero polyethylene glycol bonded substance having two or more identical bio-functional molecules and having one bio-functional molecule different from the above molecules at another end, which is a main purpose, utilization is limited.

Moreover, in the case where the introduction of two or more identical bio-functional molecules into a hetero polyethylene glycol derivative is supposed, a point largely different from the introduction of the bio-functional molecule into a usual monofunctional activated polyethylene glycol or hetero bifunctional polyethylene glycol is a fact that it is impossible to use an excess amount of the polyethylene glycol derivative relative to the bifunctional molecule for introducing the bio-functional molecule into two identical functional groups in high conversion since many kinds of impurity species are formed and rather the expensive bio-functional molecule should be allowed to react in an excess amount. Accordingly, also in view of cost, it is difficult to apply a structure having a short length between the branching point and plurality of functional groups.

From the above, in view of both performance and cost, in order to modify such a novel hetero polyethylene glycol derivative with bio-functional molecules, it is revealed that a hetero bifunctional polyethylene glycol having a sufficient distance not only between different functional groups but also between identical functional groups.

However, there has been no report of a hetero polyethylene glycol derivative having one of the two functional groups plurally, which is devised presupposing its use in medicament-related uses such as modification of such bio-functional molecules. Particularly, there is hitherto no example regarding the derivative designed also for the purpose of modifying bio-functional molecules having relatively large molecular weight, such as protein drugs and antibodies.

An object of the invention is to provide a branched hetero polyethylene glycol compound having two kinds of functional groups capable of reacting with various bio-functional molecules and having one of the functional groups plurally.

As such bio-functional molecules, there may be mentioned bio-functional molecules such as protein drugs, polypeptides, enzymes, antibodies, antibody medicaments, genes, nucleic acid compounds including oligonucleotides and the like, nucleic acid medicaments, anticancer agents, and low-molecular-weight drugs, as main ones. Also, other than these bio-functional molecules, carriers in drug delivery systems, such as liposomes and polymer micelles, and other materials and devices for diagnosis can be allowed to react. Of these, there is particularly provided a branched hetero polyethylene glycol compound most suitable for modifying bio-functional molecules including protein drugs, polypeptides, enzymes, antibodies, antibody medicaments, and the like.

Means for Solving the Problems

As a result of extensive studies for solving the above problems, the present inventors have developed a branched hetero polyethylene glycol compound having plurally one functional group of two kinds of functional groups, suitable for use in medicament-related uses including bio-functional molecules, drug carriers, and substance surfaces of base materials for diagnosis, particularly modification of bio-functional molecules having large molecular weight, including protein drugs, polypeptides, enzymes, antibodies, and antibody medicaments.

The most remarkable characteristic of the invention is a fact that plurally existing one functional group of the two kinds of the functional groups is present at an end of each polyethylene glycol chain having a certain range of the number of repeating units. Owing to this characteristic, in the case where it is considered to modify the branched hetero polyethylene glycol compound with two different kinds of bio-functional molecules, the bio-functional molecules bonded to the plural functional groups have a distance of two chains of the polyethylene glycol chain at most from each other and thus modification is achieved under the circumstances where mutual steric hindrance is reduced. Moreover, owing to the complexity of the molecular structure, the bio-functional molecules have a distance of one chain of the polyethylene glycol chain at most from the branching point which may reduce the reactivity due to steric hindrance and from another kind of bio-functional molecule to be modified at another kind of functional group.

As above, in the case where it is considered to modify the two different bio-functional molecules, owing to the structural characteristic of the branched hetero polyethylene glycol compound of the invention, the bio-functional molecule to be modified at the plurally existing one kind of functional group keeps a sufficient distance from any of three items, i.e., (1) the identical bio-functional molecule (s), (2) the branching point, and (3) the other kind of bio-functional molecule present in the same molecule. Therefore, it is possible to perform modification in higher efficiency with suppressing adverse effects resulting from steric hindrance and also a decrease in physiological activity can be suppressed.

Namely, the invention lies on the following.

(1) A branched hetero polyethylene glycol according to claim 1.
The branched hetero polyethylene glycol is represented by the formula [3]

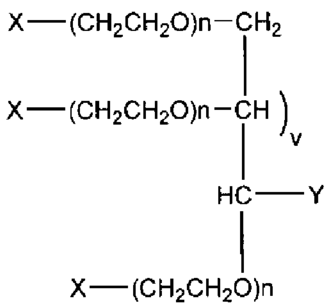

...[3]

wherein Y- = Y'-W'- ...[6];
wherein the functional group of the atomic group X and the functional group of the atomic group Y' are each selected from the group consisting of an acetal group, an aldehyde group, a maleimide group, a vinylsulfone group, an iodoacetamide group, an active ester group, an active carbonate group, a carboxyl group, an amino group, an aminoxy group, a thiol group, an allyl group, a vinyl group, an acetylene group, and an azide group; and W' is a linker moiety comprising a covalent bond such as an ester bond, a urethane bond, an amide bond, an ether bond, a carbonate bond, a saturated hydrocarbon group containing a secondary amino group, a single bond, or a saturated hydrocarbon group;
n is a number of average added moles for the polyethylene glycol chain and $20 \leq n \leq 2000$; and
v=0.

(2) The branched hetero polyethylene glycol, wherein at least one of the functional group of the atomic group X and the functional group of the atomic group Y' is selected from the group consisting of a carboxyl group, an amino group, an aminoxy group, and a thiol group.

(3) An intermediate, which is an intermediate of the branched hetero polyethylene glycol and wherein at least one of the functional group of the atomic group X and the functional group of the atomic group Y is protected by a protecting group.

(4) A polyethylene glycol bonded substance, wherein a bio-functional molecule is bonded to an end of the branched

hetero polyethylene glycol.

Advantage of the Invention

The present invention provides a branched hetero polyethylene glycol compound having one functional group of two kinds of functional groups plurally, for use in chemical modification of protein drugs, polypeptides, enzymes, antibodies, antibody medicaments, genes, nucleic acid compounds including oligonucleotides, nucleic acid medicaments, anticancer agents, other drugs such as low-molecular-weight drugs, as main one and, in addition, functionalization of drug carriers in drug delivery systems, such as liposomes and polymer micelles, and other materials and devices for diagnosis. By using the branched hetero polyethylene glycol compound, it is possible to synthesize a polyethylene glycol bonded substance having plurally one of two kinds of bio-functional molecules effectively in a state that reaction inhibition by steric repulsion at modification and reduction of functions intrinsic to the molecule, such as physiological activity after modification are suppressed.

Mode for Carrying Out the Invention

The hetero bifunctional polyethylene glycol is an end-activated polyethylene glycol compound having functional groups showing reactivity different in each of both ends of a linear polyethylene glycol chain, and different bio-functional molecules, surfaces, or the like can be introduced into the both ends.

The branched hetero polyethylene glycol in the invention has two kinds of functional groups at the ends similarly to the hetero bifunctional polyethylene glycol and the introduction of a different bio-functional molecule into each of the ends is a main purpose. Of the two kinds of functional groups, one kind thereof is plurally present and one of the bio-functional molecules to be introduced can be plurally modified.

The polyethylene glycol chain represented by $(CH_2CH_2O)n$ is a linear polymer moiety having the number n of the repeating units separately defined as follows.

The atomic group X is an atomic group which is positioned at each polyethylene glycol chain end of the branched hetero polyethylene glycol represented by the formula [1] and may contain a bonding moiety. The atomic group X is represented by the formula [5] with the functional group X' and the bonding moiety W to be bonded to the functional group X'. However, the bonding moiety W may be a single bond. In this case, the atomic group X is coincident with the functional group X'.

$$X\text{-} \quad = \quad X'\text{-}W\text{-} \qquad ...[5]$$

The bonding moiety W is a linker having a function of bonding to the polyethylene glycol chain, and is not particularly limited so long as it is a moiety comprising a covalent bond. Preferably, there may be mentioned an ester bond, a urethane bond, an amide bond, an ether bond, a carbonate bond, a saturated hydrocarbon group containing a secondary amino group, a single bond, or a saturated hydrocarbon group. The saturated hydrocarbon group has 12 or less carbon atoms and, as preferred saturated hydrocarbon groups, a methylene group, an ethylene group, a trimethylene group, a propylene group, an isopropylene group, a tetramethylene group, a butylene group, an isobutylene group, a pentamethylene group, a hexamethylene group, and the like may be mentioned.

X' is a group selected from the group consisting of an acetal group, an aldehyde group, a maleimide group, an active ester group, an active carbonate group, an amino group, an aminoxy group, an acetylene group, and an azide group. An acetal group, an aldehyde group, an active carboxylic acid, an active carbonate group, or a carboxyl group is preferred in the case of reacting with an amino group of a bio-functional molecule to be an objective substance, a maleimide group, a vinylsulfone group, an iodoacetamide group, an allyl group, or a vinyl group is preferred in the case of reacting with a thiol group of a bio-functional molecule to be an objective substance, an amino group or an aminoxy group is preferred in the case of reacting with an aldehyde group or a ketone group of a bio-functional molecule to be an objective substance, an amino group, an aminoxy group, or a thiol group is preferred in the case of reacting with a carboxyl group of a bio-functional molecule to be an objective substance, a thiol group is preferred in the case of reacting with a maleimide group of a bio-functional molecule to be an objective substance, an acetylene group is preferred in the case of reacting with an azide group of a bio-functional molecule to be an objective substance, and an azide group is preferred in the case of reacting with an acetylene group of a bio-functional molecule to be an objective substance.

The atomic group Y is an atomic group which is represented as the formula [6] with a functional group Y' and a bonding moiety W' which may be contained in the atomic group Y. Incidentally, the bonding moiety W' may be a single bond. In this case, the atomic group Y is coincident with the functional group Y'.

Y- = Y' - W'- ... [6]

The bonding moiety W' has the same definition as the bonding moiety W which may be contained in the above X. Y' is a group selected from the group consisting of an acetal group, an aldehyde group, a maleimide group, a vinylsulfone group, an iodoacetamide group, an active ester group, an active carbonate group, a carboxyl group, an amino group, an aminoxy group, a thiol group, an allyl group, a vinyl group, an acetylene group, and an azide group. Furthermore, in consideration of reaction efficiency, Y' is more preferably a group selected from the group consisting of an acetal group, an aldehyde group, a maleimide group, an active ester group, an active carbonate group, an amino group, an aminoxy group, an acetylene group, and an azide group.

The acetal group in the invention inclusive of the bonding moiety is represented by the formula [7], wherein R is a saturated hydrocarbon group having 8 or less carbon atoms. R is preferably a saturated hydrocarbon group having 4 or less carbon atoms, most preferably an ethyl group. In general, the acetal group having such a structure is a protecting group of an aldehyde group but, since deprotection of the acetal group can be conveniently performed with weak acidity, the nature at the use in the reaction with the bio-functional molecule is very close to that of the other functional groups and thus it is possible to handle the group almost similarly. Accordingly, in the invention, the acetal group is defined as a functional group having an electrophilic reactivity similarly to aldehyde.

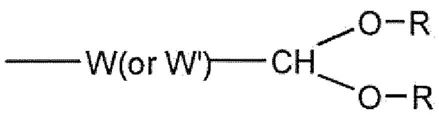
... [7]

The maleimide group in the invention inclusive of the bonding moiety is a group represented by the formula [8] and is reactive with nucleophilic groups such as a thiol group. R1 is preferably hydrogen or a methyl group, more preferably hydrogen.

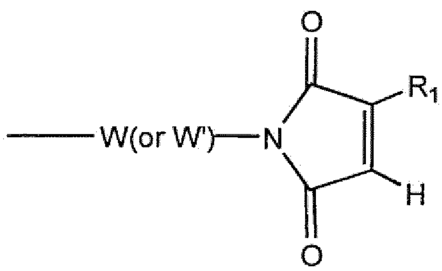
...[8]

The active ester group in the invention inclusive of the bonding moiety is a group represented by the formula [9] and is reactive with nucleophilic groups such as an amino group. R2 is preferably a phenyl group, a 3-pyridyl group, a succinimide group, a 2-benzothiazole group, or a 1-benzotriazole group, more preferably a succinimide group or a 1-benzotriazole group, and most preferably a succinimide group.

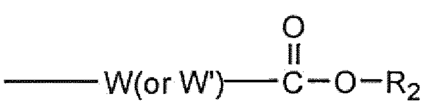
...[9]

The active carbonate group in the invention inclusive of the bonding moiety is a group represented by the formula [10] and is reactive with nucleophilic groups such as an amino group. R3 is preferably a 4-nitrophenyl group, a succinimide group, or a 1-benzotriazole group, more preferably a 4-nitrophenyl group or a succinimide group, and most preferably a succinimide group.

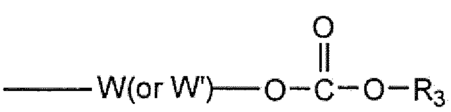
... [10]

The acetylene group in the invention inclusive of the bonding moiety is a group represented by the formula [11]

and is reactive with an azide group and the like. R4 is preferably a saturated hydrocarbon group having 8 or less carbon atoms or hydrogen, more preferably hydrogen.

$-W(\text{or } W')-C{\equiv}C-R_4$ ... [11]

The functional group contained in the atomic group X and the functional group contained in the atomic group Y should be different from each other. However, the bonding moiety W contained in the atomic group X and the bonding moiety W' contained in the atomic group Y may be the same or different from each other.

As combination of the functional group X' and the functional group Y', Y' is a group selected from a maleimide group, a vinylsulfone group, an iodoacetamide group, an active ester group, an active carbonate group, an amino group, and an aminoxy group when X' is an acetal group, Y' is a group selected from a maleimide group, a vinylsulfone group, an acetylene group, and an azide group when X' is an aldehyde group, Y' is a group selected from an acetal group, an active ester group, an active carbonate group, an acetylene group, and an azide group when X' is a maleimide group, a vinylsulfone group, or an iodoacetamide group, Y' is a group selected from an acetal group, a maleimide group, a vinylsulfone group, an iodoacetamide group, an acetylene group, and an azide group when X' is an active ester group or an active carbonate group, Y' is a group selected from an acetal group, an acetylene group, and an azide group when X' is an amino group or an aminoxy group, Y' is a group selected from an acetal group, an aldehyde group, a maleimide group, a vinylsulfone group, an iodoacetamide group, an active ester group, an active carbonate group, an amino group, and an aminoxy group when X' is an acetylene group or an azide group. Most preferably, Y' is a group selected from a maleimide group, an active ester group, an active carbonate group, an amino group, and an aminoxy group when X' is an acetal group, Y' is a group selected from an acetal group, an active ester group, an active carbonate group, an acetylene group, and an azide group when X' is a maleimide group, Y' is a group selected from an acetal group, a maleimide group, an acetylene group, and an azide group when X' is an active ester group or an active carbonate group, Y' is a group selected from an acetal group, an acetylene group, and an azide group when X' is an amino group or an aminoxy group, Y' is a group selected from an acetal group, a maleimide group, an active ester group, an active carbonate group, an amino group, and an aminoxy group when X' is an acetylene group or an azide group.

In the invention, in view of the reactivity, stability, and the like, at least one of the functional group of the atomic group X and the functional group of the atomic group Y is preferably selected from the group consisting of a carboxyl group an amino group, an aminoxy group, and a thiol group.

The branched hetero polyethylene glycol is represented by the formula [3].

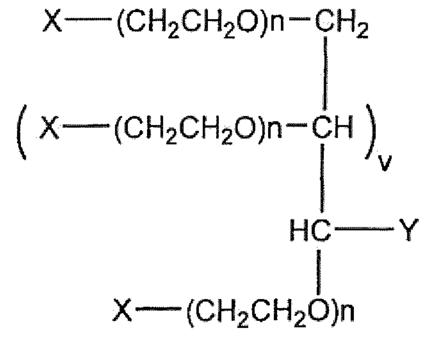

...[3]

n is the number of the repeating units per the polyethylene glycol chain having the atomic group X at the end and, in the invention, it is defined to be one calculated by various theoretical calculations based on the number-average molecular weight (Mn) of the compound. Regarding n, the most suitable setting is necessary for efficiently modifying the bio-functional molecules and thoroughly exhibiting the function. Specifically, in the case where a bio-functional molecule including a protein drug, an antibody, or the like is modified at the atomic group X and the other bio-functional molecule, surface, or the like is modified at the atomic group Y, it is necessary to keep a sufficient distance as to any of three relationships, i.e., (1) each of plural identical bio-functional molecules which are modified at the atomic group X, (2) the bio-functional molecules which are modified at the atomic group X and the branching point, and (3) the bio-functional molecules which are modified at the atomic group X and the bio-functional molecule or surface which is modified at the atomic group Y. From this viewpoint, the lower limit of n is preferably 20, more preferably 50, further preferably 80, and most preferably 120. Also, owing to difficulty in operation by an increase in solution viscosity and increase in molecular weight distribution by heterogeneity of an ethylene oxide addition reaction, the molecular weight in the branched hetero polyethylene glycol to be used in the present use should be suppressed to a certain value or less. From this viewpoint, the upper limit of n is preferably 2,000, more preferably 1,500, further preferably 1,000, and most preferably 500. By such selection of an appropriate range of n, it is possible to synthesize and use the bonded substance in a state that

reaction inhibition to be induced by steric repulsion between plurality of the bio-functional molecules which are modified at the functional groups X and Y, influence of the sterically crowded branching linker moiety E, and reduction of the functions intrinsic to the molecules, such as pharmacological activity after modification, are suppressed and further adverse effects on the reaction or the operability and quality to be induced by a viscosity increase in the use of the bonded substance and an increase of molecular weight distribution are also suppressed.

With regard to the branched hetero polyethylene glycol of the invention, as a process for introducing the polyethylene glycol chain, a coupling reaction of polyethylene glycol chains or a polymerization reaction of ethylene oxide can be selected.

As the introduction of the polyethylene glycol chain by the coupling reaction, a procedure of reacting a substrate having two amino groups such as lysine with a methoxypolyethylene glycol having, for example, an active carbonate group at the end, which is reactive to the amino group, may be mentioned as a typical example

In order to synthesize the branched hetero polyethylene glycol of the invention, it is necessary to use not a methoxypolyethylene glycol derivative but a hetero bifunctional polyethylene glycol as a polyethylene glycol. Thereafter, if necessary, a remaining end group of the two chains of the hetero bifunctional polyethylene glycol introduced and the carboxylic acid of lysine are subjected to functional group conversion. As the reaction for use in the functional group conversion, any hitherto known methods can be used but conditions not decomposing the bond formed by the coupling reaction should be appropriately selected. The bond formed by the coupling is determined by the combination of the functional groups to be used in the reaction and, in this case, is an ester bond, a urethane bond, an amide bond, a carbonate bond, a secondary amino group, or the like.

As a typical example of introducing the polyethylene glycol chain by the polymerization of ethylene oxide and subsequently introducing functional groups into the ends, the following steps may be mentioned.

(A) Polymerization of ethylene oxide

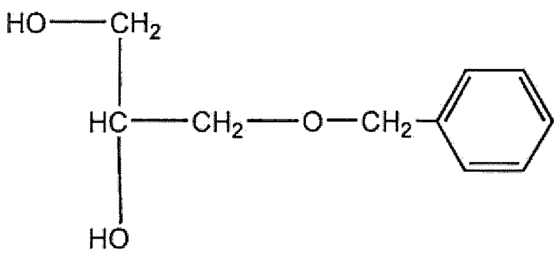

[14]

Ethylene oxide is polymerized to the remaining two hydroxyl groups of the compound of the formula [14] where the hydroxyl group at 3-position of glycerin has been substituted with a benzyl group, in an amount of 40 to 4,000 molar equivalents to the compound of the formula [14] in toluene or without any solvent under an alkali condition of metal sodium or metal potassium, sodium hydride, potassium hydride, sodium methoxide, potassium t-butoxide, or the like to obtain a branched hetero polyethylene glycol of the formula [15].

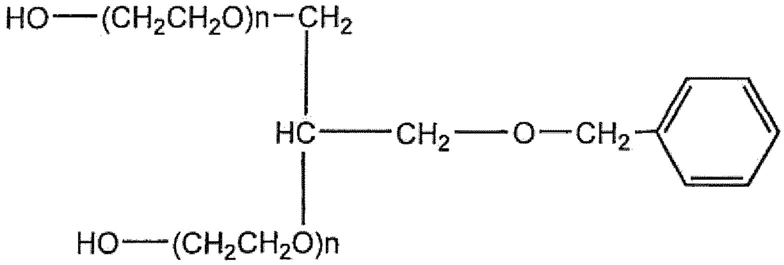

[15]

In the formula, the branching moiety corresponding to E of the formula [1] is the same as one defined in the formula [2]. The branching moiety to be used in the polymerization step of ethylene oxide is preferably a stable group under such an alkali condition, more preferably a saturated hydrocarbon group or a saturated hydrocarbon group containing an ether bond. As the skeleton of the branching moiety, most preferably, a glycerin moiety, a xylitol moiety, and the like may be mentioned. Moreover, instead of a benzyl group, the other protecting group can be used so long as it is a stable group under the above alkali condition. For example, there may be used a tetrahydropyranyl group, a t-butyl

group, and the like in the case of a protecting group for a hydroxyl group and an acetal group such as a diethyl acetal group in the case of a protecting group for an aldehyde group.

(B) Functional group conversion of hydroxyl group at polyethylene glycol chain end

Subsequently, after toluene is added to the branched polyethylene glycol compound of the formula [15] and is refluxed under ordinary pressure to achieve removal of water, an excess amount of methanesulfonyl chloride is added in the presence of a base such as triethylamine to form a mesylate. This compound is allowed to react with a sodium alcoholate compound of 3,3-diethoxy-1-propanol to introduce a diethoxy acetal group, thereby a compound of the formula [16] being obtained.

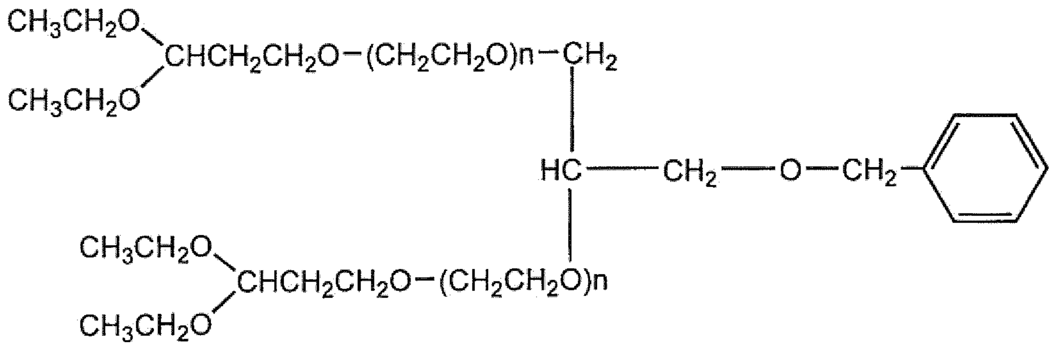

[16]

Other than the acetal group, it is possible to introduce various protecting groups and functional groups into ends of polyethylene glycol chains. However, in the formula [16], a point to be noted is a fact that it is necessary to perform functional group conversion of the hydroxyl group under conditions where the protecting group in the vicinity of the branching moiety that is a benzyl group in this case can be stably present. For example, it is necessary that such a reaction condition that it does not become reductive one is selected in the case of using a benzyl group as a protecting group, and such a reaction condition that it does not become acidic one is selected in the case of using a tetrahydropyranyl group, a diethyl acetal group, or the like.

(C) Deprotection of protecting group in vicinity of branching moiety

Next, catalytic reduction of the compound of the formula [16] is carried out in the presence of a reduction catalyst such as Pd/C and a hydrogen donor such as hydrogen gas or cyclohexene to achieve a debenzylation reaction, thereby a compound of the formula [17] being obtained.

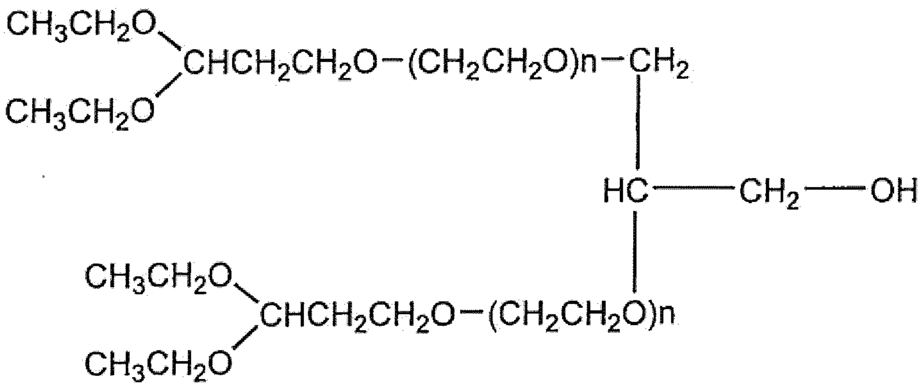

[17]

On this occasion, the functional group/protecting group introduced into the polyethylene glycol chain end should be a stable group under the deprotection condition for the deprotection of the protecting group in the vicinity of the branching moiety, as a result of the functional group conversion at one time or plurality of times as in the above step (B).

For example, as a group to be introduced into the polyethylene glycol chain, it is necessary to select a stable protecting group/functional group against hydrogen reduction that is a deprotection condition, such as an acetal group, a tetrahydropyranyl group, a phthalimide group, an oxyphthalimide group, or a thiol group, in the case of using a benzyl

group as a protecting group of the hydroxyl group in the vicinity of the branching moiety; a stable protecting group/functional group against a weakly acidic condition that is a deprotection condition, such as a maleimide group, a carboxyl group, an amino group, an aminoxy group, or a thiol group, in the case of using a tetrahydropyranyl group as a protecting group of the hydroxyl group or an acetal group or the like as a protecting group of an aldehyde group; a stable protecting group/functional group against a strongly acidic condition that is a deprotection condition, such as a carboxyl group, an amino group, an aminoxy group, or a thiol group, in the case of using a t-butoxy group or the like. However, since enormous numbers of reactions are present for the functional group conversion and subsequent further functional group conversion is possible, this description does not limit the finally obtained functional group species at the end.

Incidentally, in the case where an acetal group such as a diethyl acetal group that is a protecting group of an aldehyde is used as a protecting group in the vicinity of the branching moiety, since it is possible to use the group as it is for deprotection and reaction in the reaction system as mentioned above, it is not always necessary to perform deprotection at this stage.

(D) Functional group conversion in vicinity of branching moiety

Next, the compound of the formula [17] is subjected to functional group conversion to introduce various functional groups as follows. After toluene is added and is refluxed under ordinary pressure to achieve removal of water, an excess amount of methanesulfonyl chloride is added in the presence of triethylamine to form a mesylate. Then, this compound is allowed to react with ammonia to introduce an amino group, thereby a compound of the formula [18] being obtained.

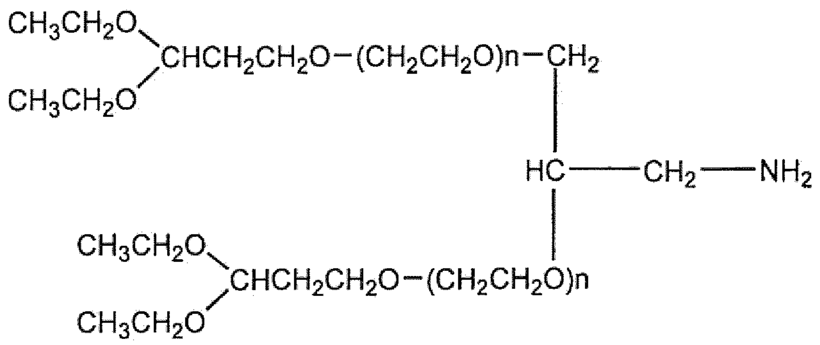

[18]

Further, the compound of the formula [18] is allowed to react with N-succinimidylmaleimidopropionic acid in the presence of a base such as N-methylmorpholine to introduce a maleimide group, thereby a compound of the formula [19] being obtained.

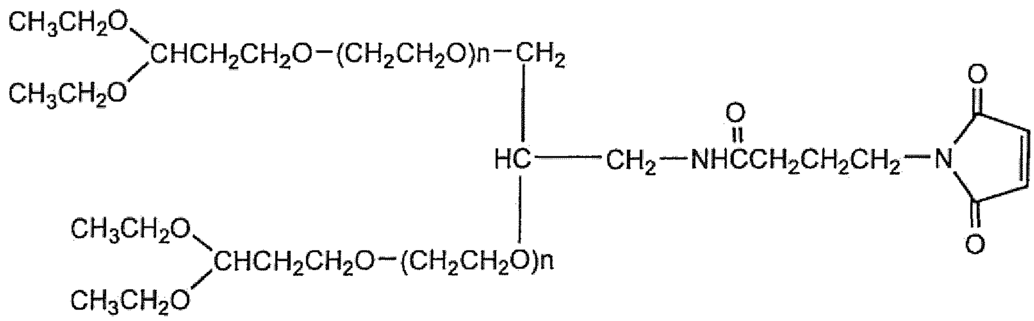

[19]

The branched hetero polyethylene glycol having plurality of functional groups at polyethylene glycol chain ends and a functional group in the vicinity of the branching moiety introduced thereinto as the formulae [18] and [19] can be used for the reactions with bio-functional molecules.

(E) Functional group conversion/deprotection at polyethylene glycol chain ends

The branched hetero polyethylene glycol represented by the formula [18] or [19] can be used for the reaction with bio-functional molecules in this state. However, for example, regarding the compound of the formula [19], by selecting an appropriate acidic condition, it is also possible to perform deprotection with leaving the maleimide group to convert

the acetal group to an aldehyde group.

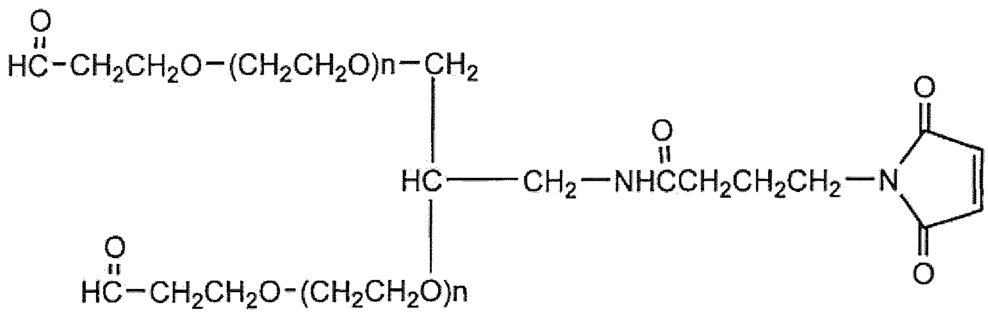

[20]

It is also possible to repeat the steps of functional group conversion such as the above (D) and (E) several times for the purpose of introduction of respective arbitrary functional groups and it is possible to introduce various kinds of functional groups by performing functional group conversion under aforementioned conditions.

The objective substances to be bonded to the branched hetero polyethylene glycol of the invention are bio-functional molecules including protein drugs, polypeptides, enzymes, antibodies, antibody medicaments, genes, nucleic acid compounds including oligonucleotides, nucleic acid medicaments, anticancer agents, the other drugs such as low-molecular-weight drugs or the other molecules, or carriers in drug delivery systems, such as liposomes and polymer micelles, and other materials and devices for diagnosis . As a typical example, there may be mentioned an example where a targeting molecule such as an antibody or a peptide ligand is introduced into the atomic group X and a drug such as an anticancer agent or a protein drug or a carrier containing a drug included therein, such as a liposome or a polymer micelle, is bonded to another atomic group Y. In this case, it is expected to remarkably enhance migration ability of the drug or carrier to an objective tissue as compared with the case of using a conventional hetero bifunctional polyethylene glycol where one targeting molecule is bonded. As another example, there may be mentioned an example where a drug is introduced into the atomic group X and a targeting molecule is introduced into another atomic group Y. In this case, a large amount of the drug can be introduced and thus it is expected to enhance the amount of the drug to be transported as compared with the case of using the hetero bifunctional polyethylene glycol. The bio-functional molecules to be reacted and introduced into the atomic group X and the atomic group Y are not particularly limited. However, when the number n of the repeating units per the polyethylene glycol chain having the atomic group X and the structural characteristic of the branched hetero polyethylene glycol of the invention are considered, a larger advantage can be obtained by using a bio-functional molecule having a molecular weight of 2,000 or more, further preferably 3,000 or more for the atomic group X. Also, there is no particular limitation but, form the same viewpoint, a larger advantage can be obtained by using a bio-functional molecule having a molecular weight of 3,000 or less, further preferably 2,000 or less for the atomic group Y.

## Examples

The following will describe the invention in further detail with reference to Examples.

The molecular weight and molecular weight distribution of polyethylene glycol compounds including the branched hetero polyethylene glycol or intermediates thereof were determined by analysis on gel permeation chromatography (GPC). In the invention, measurement was performed using SHODEX GPC SYSTEM-11 as a GPC system and SHODEX RIX8 as a differential refractometer that was a detector, connecting three columns of SHODEX KF801L, KF803L, and KF804L (ϕ 8mn × 300 mm) serially, setting the temperature of the column oven to 40°C, using tetrahydrofuran as an eluent, setting the flow rate to 1 ml/minute, setting the concentration of a sample to 0.1% by mass, and setting an injection volume to 0.1 ml. As a calibration curve, a curve prepared using ethylene glycol, diethylene glycol, and triethylene glycol manufactured by Kanto Chemical Co., Inc. and Polymer Standards for GPC of polyethylene glycols or polyethylene oxides each having a molecular weight of 600 to 70,000 manufactured by Polymer Laboratory was used. For data analysis, BORWIN GPC calculation program was used. Mn indicates number-average molecular weight, Mw indicates weight-average molecular weight, and Mp indicates peak-top molecular weight. As for the molecular weight distribution, a calculated value thereof was shown as Mw/Mn.

For $^{1}$H-NMR analysis, JNM-ECP400 or JNM-ECA600 manufactured by JEOL DATUM Ltd. was used. Measurement was performed using $CDCl_3$ as a deuterated solvent, using a ϕ 5 mm tube, and using TMS as an internal standard substance.

(Example 1)

To a 300 ml four-neck flask equipped with a thermometer, a nitrogen-inlet tube, and a stirrer were added 18.2 g (0.1 mol) of 3-benzyloxy-1,2-propanediol, 150 g of anhydrous toluene, 0.9 g (39 mmol: 26% by mol) of metal sodium, and the whole was stirred at room temperature with introducing nitrogen until metal sodium was dissolved. The solution was charged into a 5 L autoclave and, after inside of the system was replaced with nitrogen, the temperature was raised to 100°C. After 1,982 g (45 mol) of ethylene oxide was added thereto at 100 to 130°C under a pressure of 1 MPa or less, the reaction was further continued for 2 hours. After unreacted ethylene oxide gas was removed under reduced pressure, the whole was cooled to 60°C and pH was adjusted to 7.5 with an 85% aqueous phosphoric acid solution to obtain the following compound (a1).
$^1$H-NMR; δ (ppm):

3.40-3.90 (1785H, m, $HO(CH_2CH_2O)_n$-$CH_2$, $HO(CH_2CH_2O)_n$-$CH$, $CH_2O$-$CH_2Ph$), 4.54 (2H, s, -$CH_2Ph$), 7.27-7.38 (5H, m, -$CH_2Ph$) GPC analysis;
number-average molecular weight (Mn): 19,654, weight-average molecular weight (Mw): 20,285, polydispersity (Mw/Mn): 1.032

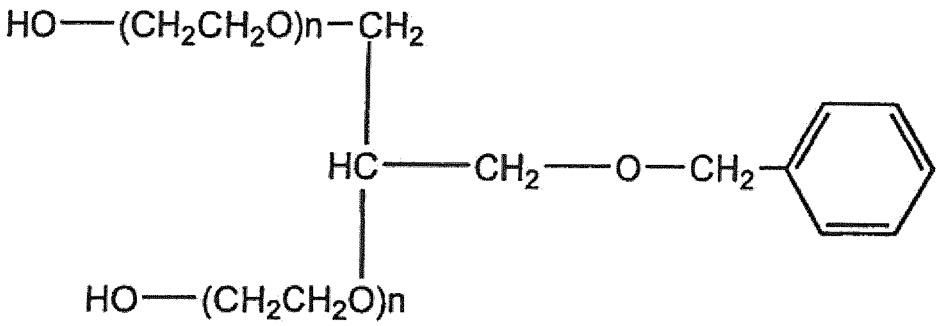

(a1) n = about 221

(Example 2)

Into a 3 L four-neck flask fitted with a thermometer, a nitrogen-inlet tube, a stirrer, a Dean-stark tube, and a cooling tube were charged 372 g (18.6 mmol) of the above compound (a1) and 1,860 g of toluene. The whole was heated under reflux and water was removed as an azeotrope. After cooling to room temperature, 6.02 g (59.5 mmol) of triethylamine and 5.54 g (48.4 mmol) of methanesulfonyl chloride were added thereto and allowed to react at 40°C for 3 hours. Subsequently, a toluene (256.8 g) solution of 85.6 g (465 mmol) of 3,3-diethoxy-1-propanol containing 2.58 g (112 mmol) of sodium dissolved therein was added thereto, followed by reaction at 70°C for 5 hours. After filtration of the reaction solution, the filtrate was transferred to a 10 L stainless steel pot and crystallization was performed with adding 1.488 g of ethyl acetate, 1, 488 g of ethanol, and 2, 976 g of hexane. After the precipitated crystals were filtrated to remove the solvents, the crystals were transferred to a 10 L stainless steel pot. After 1,488 g of ethyl acetate and 1,488 g of ethanol were added thereto and heated to dissolve the crystals at 40°C, the solution was cooled to 20°C and crystallization was performed with adding 2.976 g of hexane. Thereafter, similar crystallization was repeated three times and, after washing with hexane, the crystals were collected by filtration and dried to obtain the following compound (a2).
1H-NMR: δ (ppm):
1.16-1.24 (12H, t, $(CH_3CH_2O)_2$-$CHCH_2CH_2$-), 1.85-1.95 (4H, q, $(CH_3CH_2O)_2$-$CHCH_2CH_2$-), 3.40-3.90 (1725H, m, -$(CH_2CH_2O)_n$-$CH_2$, -$(CH_2CH_2O)_n$-$CH$, $CH_2O$-$CH_2Ph$, $(CH_3CH_2O)_2$-$CHCH_2CH_2$-), 4.54 (2H, s, -$CH_2Ph$), 4.60-4.68 (2H, t, $(CH_3CH_2O)_2$-$CHCH_2CH_2$-), 7.27-7.38 (5H, m, -$CH_2Ph$)
GPC analysis;
number-average molecular weight (Mn): 19,190, weight-average molecular weight (Mw): 19,496, polydispersity (Mw/Mn): 1.016

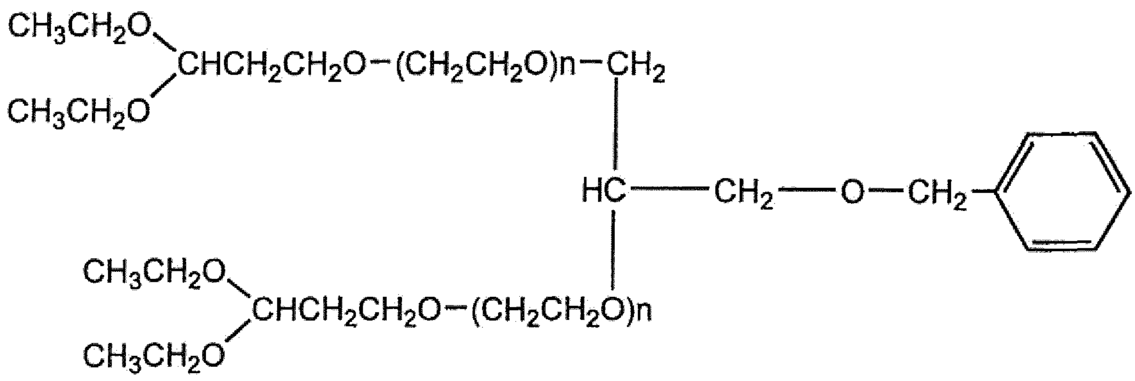

(a2) n = about 213

(Example 3)

Into a 1 L four-neck flask fitted with a thermometer, a nitrogen-inlet tube, a stirrer, and a cooling tube were charged 50 g of the above compound (a2) and 25 g of 5% palladium-carbon (a 50% hydrous product). After replacement with nitrogen, 400 g of methanol and 67 g of cyclohexene were added thereto, the temperature was raised, and gentle refluxing was performed at 52 to 55°C to effect reaction for 2 hours. After cooling of the reaction solution to room temperature, palladium-carbon was filtrated off and the filtrate was concentrated. To the concentrate were added 400 g of toluene and 200 g of hexane, and crystallization was performed. The resulting crystals were collected by filtration and dried to obtain the following compound (a3).
$^1$H-NMR; δ (ppm):
1.16-1.24 (12H, t, $(CH_3CH_2O)_2$-$CHCH_2CH_2$-), 1.85-1.95 (4H, q, $(CH_3CH_2O)_2$-$CHCH_2CH_2$-), 3.40-3.90 (1673H, m, -$(CH_2CH_2O)_n$-$CH_2$, -$(CH_2CH_2O)_n$-CH, $CH_2$OH, $(CH_3CH_2O)_2$-$CHCH_2CH_2$-), 4.60-4.68 (2H, t, $(CH_3CH_2O)_2$-$CHCH_2CH_2$-)
GPC analysis;
number-average molecular weight (Mn): 18,573, weight-average molecular weight (Mw): 19,123, polydispersity (Mw/Mn): 1.030

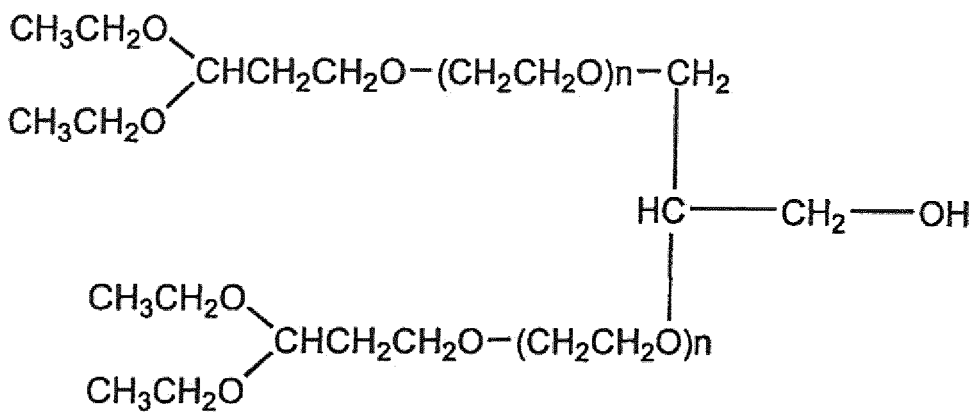

(a3) n = about 207

(Example 4)

Into a 200 mL three-neck flask fitted with a thermometer, a nitrogen-inlet tube, a stirrer, a Dean-stark tube, and a cooling tube were charged 13.1 g (0.66 mmol) of the above compound (a3) and 79 g of toluene. The whole was heated under reflux and water was removed as an azeotrope. After cooling to room temperature, 0.33 g (3.3 mmol) of triethylamine and 0.30 g (2.6 mmol) of methanesulfonyl chloride were added thereto and allowed to react at 40°C for 3 hours. After filtration of the reaction solution, the filtrate was transferred to a 300 mL beaker and crystallization was performed with adding 100 g of ethyl acetate and 50 g of hexane. After the precipitated crystals were filtrated to remove the solvents, the crystals were transferred to a 10 L stainless steel pot. After 1,488 g of ethyl acetate and 1,488 g of ethanol were added thereto and heated to dissolve the crystals at 40°C, the solution was cooled to 20°C and crystallization was performed with adding 2, 976 g of hexane. Thereafter, similar crystallization was repeated three times and, after washing

with hexane, the crystals were collected by filtration and dried to obtain the following compound (a4).
$^1$H-NMR; δ (ppm) :
1.16-1.24 (12H, t, $(CH_3CH_2O)_2$-$CHCH_2CH_2$-), 1.85-1.95 (4H, q, $(CH_3CH_2O)_2$-$CHCH_2CH_2$-), 3.08 (3H, s, -$OSO_2CH_3$), 3.40-3.90 (1732H, m, -$(CH_2CH_2O)_n$-$CH_2$, -$(CH_2CH_2O)_n$-CH, $CH_2OH$, $(CH_3CH_2O)_2$-$CHCH_2CH_2$-), 4.24-4.44 (2H, m, -$CH_2O$-$OSO_2CH_3$), 4.60-4.68 (2H, t, $(CH_3CH_2O)_2$-$CHCH_2CH_2$-)
GPC analysis;
number-average molecular weight (Mn): 19,274, weight-average molecular weight (Mw): 20,046, polydispersity (Mw/Mn): 1.040

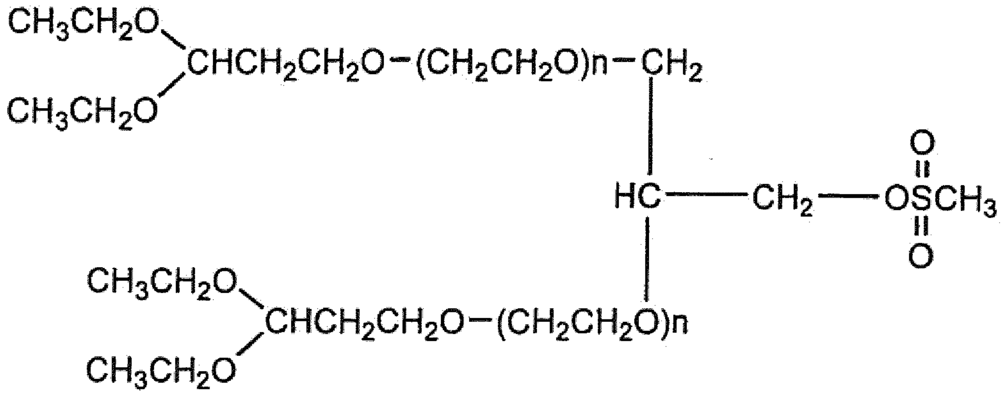

(a4) n = about 214

(Example 5)

Into a 100 mL three-neck flask fitted with a thermometer, a nitrogen-inlet tube, a stirrer, a Dean-stark tube, and a cooling tube were charged 10.0 g (0.50 mmol) of the above compound (a4), 80 g of ammonia water, and 20 g of ion-exchange water, followed by reaction at 55°C for 8 hours. Ammonia was removed at 55°C under slightly reduced pressure for 10 hours, followed by extraction with adding 100 g of chloroform. After the chloroform solution was concentrated, 52 g of toluene was added thereto and the concentrate was dissolved at 40°C, followed by filtration. Thereafter, crystallization was performed with adding 30 g of hexane at room temperature in a 200 mL beaker. After the precipitated crystals were filtrated to remove the solvents, the crystals were washed with hexane, collected by filtration, and dried to obtain the following compound (a5).
$^1$H-NMR; δ (ppm):
1.16-1.24 (12H, t, $(CH_3CH_2O)_2$-$CHCH_2CH_2$-), 1.85-1.95 (4H, q, $(CH_3CH_2O)_2$-$CHCH_2CH_2$-), 2.70-2.95 (2H, m, -$CH_2NH_2$), 3.40-3.90 (1730H, m, -$(CH_2CH_2O)_n$-$CH_2$, -$(CH_2CH_2O)_n$-CH, $CH_2OH$, $(CH_3CH_2O)_2$-$CHCH_2CH_2$-), 4.60-4.68 (2H, t, $(CH_3CH_2O)_2$-$CHCH_2CH_2$-)
GPC analysis;
number-average molecular weight (Mn): 19,217, weight-average molecular weight (Mw): 20,362, polydispersity (Mw/Mn): 1.060

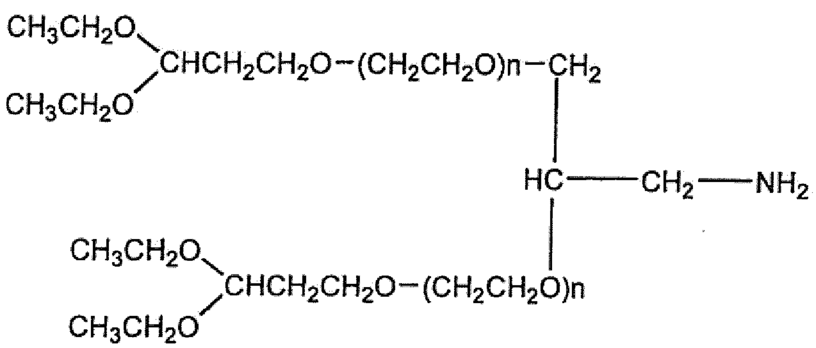

(a5) n = about 214

(Example 6)

Into a 100 mL three-neck flask fitted with a thermometer, a nitrogen-inlet tube, a stirrer, a Dean-stark tube, and a cooling tube were charged 5.0 g (0.25 mmol) of the above compound (a5), 26 g of toluene, 4.0 g of acetonitrile, N-methylmorpholine (1.25 mmol), and N-succinimidyl 3-maleimidopropionate (0.375 mmol), followed by reaction at room temperature for 4 hours. After the solution was filtrated, crystallization was performed with adding 100 g of ethyl acetate and 50 g of hexane at room temperature in a 300 mL beaker. Then, after 100 g of ethyl acetate was added and the crystals were dissolved at 40°C, the solution was cooled to room temperature and 100 g of hexane was added to precipitate crystals. After the crystallization was repeated further twice, the crystals were washed with hexane, collected by filtration, and dried to obtain the following compound (a6).
$^1$H-NMR; δ (ppm):
1.16-1.24 (12H, t, $(CH_3CH_2O)_2$-$CHCH_2CH_2$-), 1.85-1.95 (4H, q, $(CH_3CH_2O)_2$-$CHCH_2CH_2$-), 2.51 (2H, t, $NHCOCH_2CH_2$), 3.40-3.90 (1736H, m, -$(CH_2CH_2O)_n$-$CH_2$, -$(CH_2CH_2O)_n$-CH, $CH_2OH$, $(CH_3CH_2O)_2$-$CHCH_2CH_2$-, $CH_2NHCOCH_2CH_2$), 4.60-4.68 (2H, t, $(CH_3CH_2O)_2$-$CHCH_2CH_2$-), 6.70 (2H, s, CH=CH)
GPC analysis;
number-average molecular weight (Mn): 19,395, weight-average molecular weight (Mw): 20,600, polydispersity (Mw/Mn): 1.062

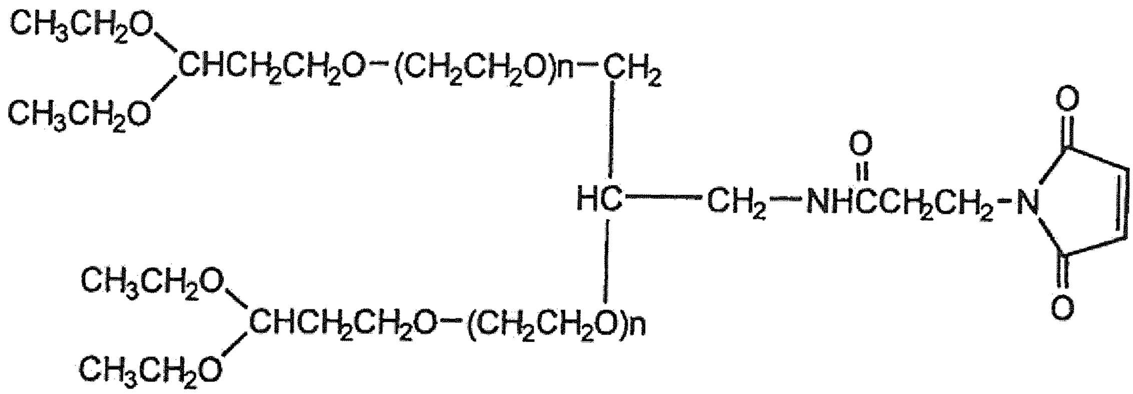

(a6) n = about 215

(Example 7)

Into a 200 mL beaker fitted with a thermometer, a nitrogen-inlet tube, and a stirring bar were charged 2.0 g (0.10 mmol) of the above compound (a5) and 40 g of ion-exchange water, followed by dissolution. After the solution was prepared to be pH 2 with hydrochloric acid and stirred at room temperature for 2 hours, the solution was neutralized to pH 6 with an aqueous sodium hydroxide solution, followed by extraction with adding 100 g of chloroform. After the chloroform solution was concentrated, 40 g of toluene was added thereto and the concentrate was dissolved at 40°C, followed by filtration. Thereafter, crystallization was performed with adding 20 g of hexane at room temperature in a 200 mL beaker. After the precipitated crystals were filtrated to remove the solvents, the crystals were washed with hexane, collected by filtration, and dried to obtain the following compound (a7) .
$^1$H-NMR; δ (ppm):
2.51 (2H, t, $NHCOCH_2CH_2$), 2.63-2.73 (4H, HCO-$CH_2CH_2$-), 3.40-3.90 (1735H, m, -$(CH_2CH_2O)_n$-$CH_2$, -$(CH_2CH_2O)_n$-CH, HCO-$CH_2CH_2$-, $CH_2NHCOCH_2CH_2$), 6.70 (2H, s, CH=CH), 9.80 (2H, HCO-$CH_2CH_2$-)
GPC analysis;
number-average molecular weight (Mn): 19,319, weight-average molecular weight (Mw): 20,634, polydispersity (Mw/Mn): 1.068

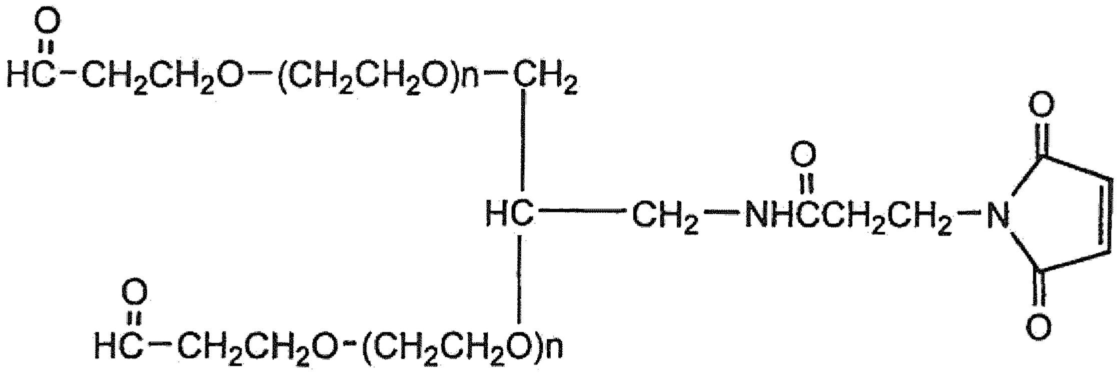

(a7) n = about 216

(Example 8)

Into a 1 L four-neck flask fitted with a thermometer, a nitrogen-inlet tube, a stirrer, a Dean-stark tube, and a cooling tube were charged 200 g (10.0 mmol) of the above compound (a1) and 600 g of toluene. The whole was heated under reflux and water was removed as an azeotrope. After cooling to 40°C, 1,000 g of anhydrous chloroform, 8.83 g (60.0 mmol) of phthalimide and 15.7 g (60.0 mmol) of triphenylphosphine were added thereto, followed by stirring and dissolution. After cooling to room temperature, 12.1 g (60.0 mmol) of diisopropylazodicarboxylate was allowed to react at 40°C for 3 hours. Subsequently, a toluene (256.8 g) solution of 85.6 g (465 mmol) of 3,3-diethoxy-1-propanol containing 2.58 g (112 mmol) of sodium dissolved therein was added thereto and allowed to react at 70°C for 5 hours. After filtration of the reaction solution, the filtrate was transferred to a 10 L stainless steel pot and crystallization was performed with adding 1,488 g of ethyl acetate, 1,488 g of ethanol, and 2,976 g of hexane. After the precipitated crystals were filtrated to remove the solvents, the crystals were transferred to a 10 L stainless steel pot. After 1,488 g of ethyl acetate and 1,488 g of ethanol were added thereto and heated to dissolve the crystals at 40°C, the solution was cooled to 20°C and crystallization was performed with adding 2,976 g of hexane. Thereafter, similar crystallization was repeated three times and, after washing with hexane, the crystals were collected by filtration and dried to obtain the following compound (a8).
$^1$H-NMR; $\delta$ (ppm) :
3.40-3.90 (1728H, m, $-(CH_2CH_2O)_n-CH_2$, $-(CH_2CH_2O)_n-CH$, $CH_2O-CH_2Ph$), 4.54 (2H, s, $-CH_2Ph$), 7.27-7.38 (5H, m, $-CH_2Ph$), 7.65-7.95 (4H, m, $Ph(CO)_2N-$)
GPC analysis;
number-average molecular weight (Mn): 19,398, weight-average molecular weight (Mw): 19,824, polydispersity (Mw/Mn): 1.022

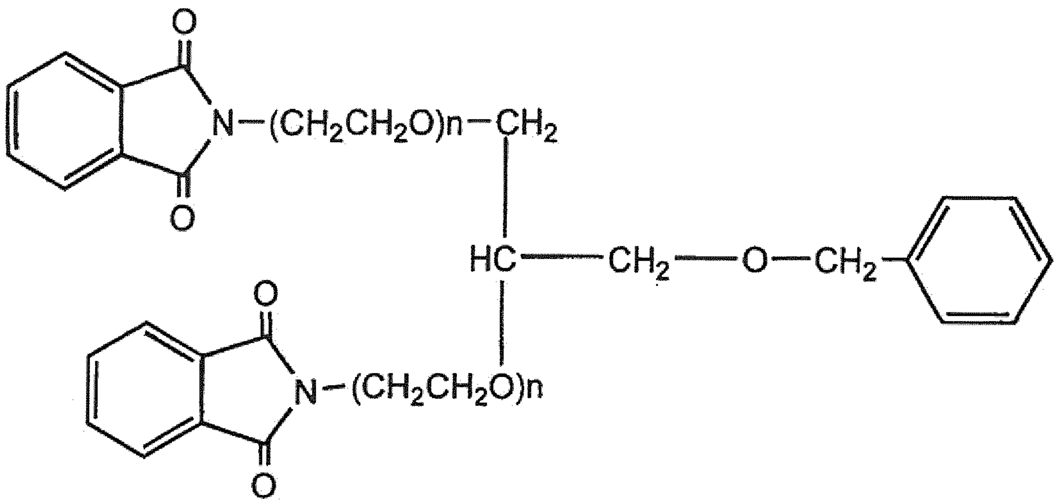

(a8) n = about 215

(Example 9)

Into a 1 L four-neck flask fitted with a thermometer, a nitrogen-inlet tube, a stirrer, and a cooling tube were charged 50 g of the above compound (a8) and 25 g of 5% palladium-carbon (a 50% hydrous product) . After replacement with nitrogen, 500 g of methanol and 68 g of cyclohexene were added thereto, the temperature was raised, and gentle refluxing was performed at 52 to 55°C to effect reaction for 5 hours. After cooling of the reaction solution to room temperature, palladium-carbon was filtrated off and the filtrate was concentrated. To the concentrate were added 400 g of toluene and 200 g of hexane, and crystallization was performed. The resulting crystals were collected by filtration and dried to obtain the following compound (a9) .

$^1$H-NMR; δ (ppm):

3.40-3.90 (1684H, m, $-(\underline{CH_2CH_2}O)_n-\underline{CH_2}$, $-(\underline{CH_2CH_2}O)_n-\underline{CH}$, $\underline{CH_2}OH$), 7.65-7.95 (4H, m, $\underline{Ph}(CO)_2N-$)

GPC analysis;

number-average molecular weight (Mn): 18,817, weight-average molecular weight (Mw): 19,171, polydispersity (Mw/Mn): 1.019

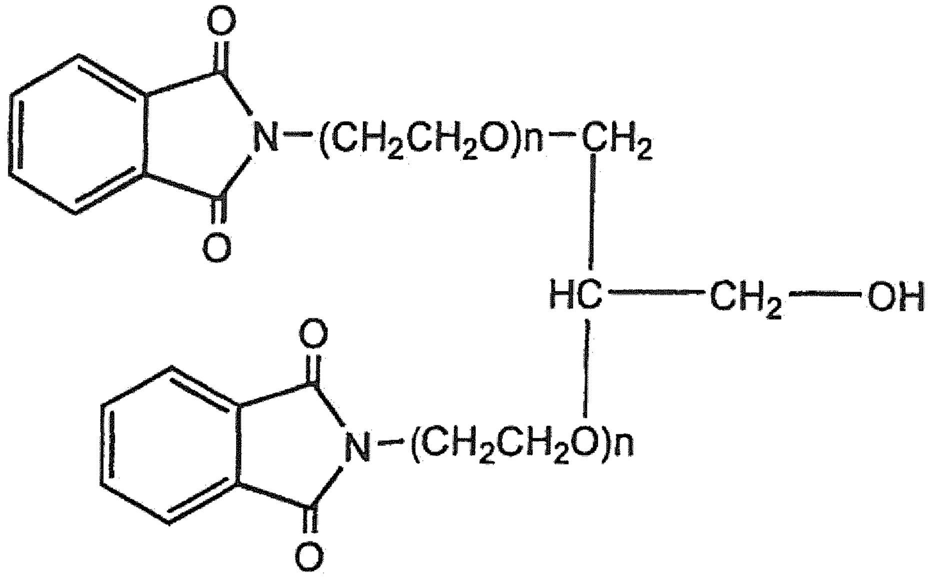

(a9) n = about 210

(Example 10)

Into a 1 L four-neck flask fitted with a thermometer, a nitrogen-inlet tube, a stirrer, a Dean-stark tube, and a cooling tube were charged 200 g (10.0 mmol) of the above compound (a1) and 600 g of toluene. The whole was heated under reflux and water was removed as an azeotrope. After cooling to 40°C, 1,000 g of anhydrous chloroform, 8.83 g (60.0 mmol) of phthalimide and 15.7 g (60.0 mmol) of triphenylphosphine were added thereto, followed by stirring and dissolution. After cooling to room temperature, 12.1 g (60.0 mmol) of diisopropylazodicarboxylate was allowed to react at 40°C for 3 hours. Subsequently, a toluene (256.8 g) solution of 85.6 g (465 mmol) of 3,3-diethoxy-1-propanol containing 2.58 g (112 mmol) of sodium dissolved therein was added thereto and allowed to react at 70°C for 5 hours. After filtration of the reaction solution, the filtrate was transferred to a 10 L stainless steel pot and crystallization was performed with adding 1,488 g of ethyl acetate, 1,488 g of ethanol, and 2,976 g of hexane. After the precipitated crystals were filtrated to remove the solvents, the crystals were transferred to a 10 L stainless steel pot. After 1,488 g of ethyl acetate and 1,488 g of ethanol were added thereto and heated to dissolve the crystals at 40°C, the solution was cooled to 20°C and crystallization was performed with adding 2,976 g of hexane. Thereafter, similar crystallization was repeated three times and, after washing with hexane, the crystals were collected by filtration and dried to obtain the following compound (a10).

$^1$H-NMR; δ (ppm):

2.83-2.89 (4H, t, $NH_2-\underline{CH_2}CH_2-$), 3.40-3.90 (1676H, m, $-(\underline{CH_2CH_2}O)_n-\underline{CH_2}$, $(\underline{CH_2CH_2}O)_n-\underline{CH}$, $\underline{CH_2}OH$)

GPC analysis;

number-average molecular weight (Mn): 18,559, weight-average molecular weight (Mw): 19,264, polydispersity (Mw/Mn): 1.038

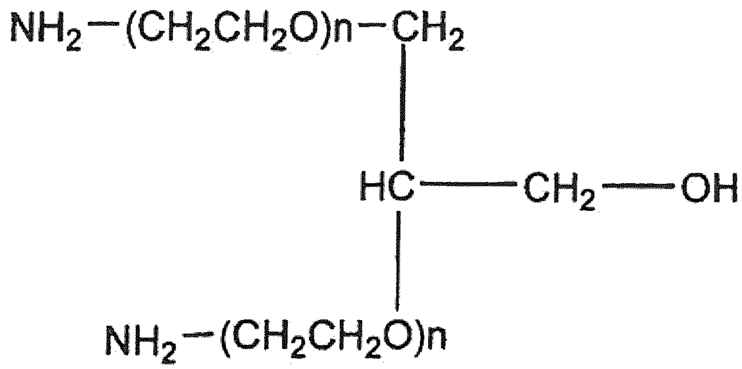

(a10) n = about 210

(Example 11)

Into a 100 mL three-neck flask fitted with a thermometer, a nitrogen-inlet tube, a stirrer, a Dean-stark tube, and a cooling tube were charged 5.0 g (0.25 mmol) of the above compound (a5), 26 g of toluene, 4.0 g of acetonitrile, N-methylmorpholine (2.50 mmol), and N-succinimidyl 3-maleimidopropionate (0.75 mmol), followed by reaction at room temperature for 4 hours. After the solution was filtrated, crystallization was performed with adding 100 g of ethyl acetate and 50 g of hexane at room temperature in a 300 mL beaker. Then, after 100 g of ethyl acetate was added and the crystals were dissolved at 40°C, the solution was cooled to room temperature and 100 g of hexane was added to precipitate crystals. After the crystallization was repeated further twice, the crystals were washed with hexane, collected by filtration, and dried to obtain the following compound (a11).
$^1$H-NMR; δ (ppm):
2.51 (2H, t, $CH_2CH_2CONH$), 3.40-3.90 (1767H, m, $-(CH_2CH_2O)_n-CH_2$, $-(CH_2CH_2O)_n-CH$, $CH_2OH$, $CH_2CH_2CONH$), 6.50 (1H, s, $CH_2CH_2CONH$), 6.70 (2H, s, $CH{=}CH$)
GPC analysis;
number-average molecular weight (Mn): 19,734, weight-average molecular weight (Mw): 21,336, polydispersity (Mw/Mn): 1.081

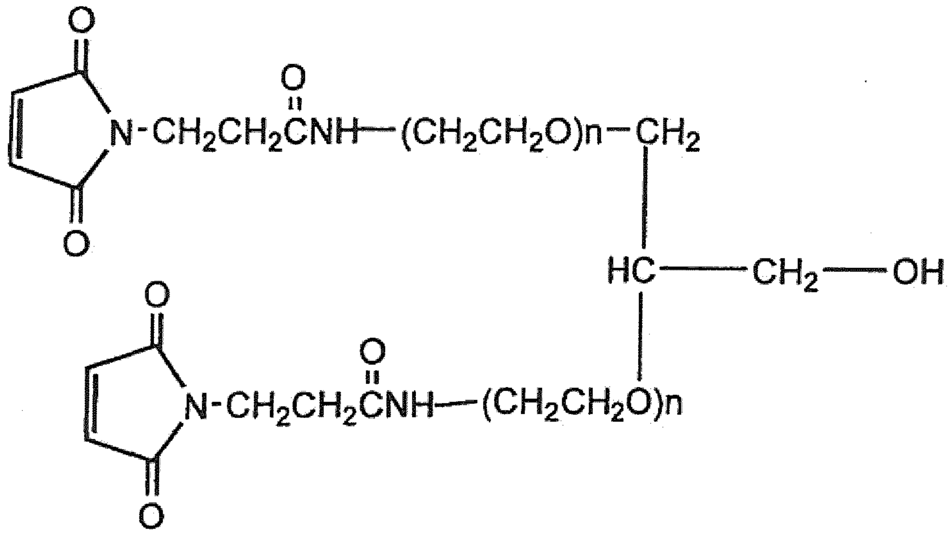

(a11) n = about 220

(Example 12)

Into a 100 mL three-neck flask fitted with a thermometer, a nitrogen-inlet tube, a stirrer, a Dean-stark tube, and a cooling tube were charged 4.0 g (0.20 mmol) of the above compound (a5), 20 g of dichloromethane, triethylamine (2.50 mmol), and N,N-disuccinimidyl carbonate (0.75 mmol), followed by reaction at room temperature for 4 hours. After the solution was filtrated and concentrated, crystallization was performed with adding 100 g of ethyl acetate and 50 g

of hexane in a 300 mL beaker. Then, after 100 g of ethyl acetate was added and the crystals were dissolved at 40°C, the solution was cooled to room temperature and 100 g of hexane was added to precipitate crystals. After the crystallization was repeated further three times, the crystals were washed with hexane, collected by filtration, and dried to obtain the following compound (a12).

$^1$H-NMR: δ (ppm):

2.51 (2H, t, $CH_2CH_2CONH$), 2.84 (4H, s, $-CH_2O$-COO-succinimide), 3.40-3.90 (1751H, m, $-(CH_2CH_2O)_n-CH_2$, $-(CH_2CH_2O)_n$-CH, $-CH_2O$-COO-succinimide, $CH_2CH_2CONH$), 4.36-4.52 (2H, m, $-CH_2O$-COO-succinimide), 6.37 (1H, s, $CH_2CH_2CONH$), 6.70 (2H, s, CH=CH)

GPC analysis;

number-average molecular weight (Mn): 19,659, weight-average molecular weight (Mw): 21,450, polydispersity (Mw/Mn): 1.091

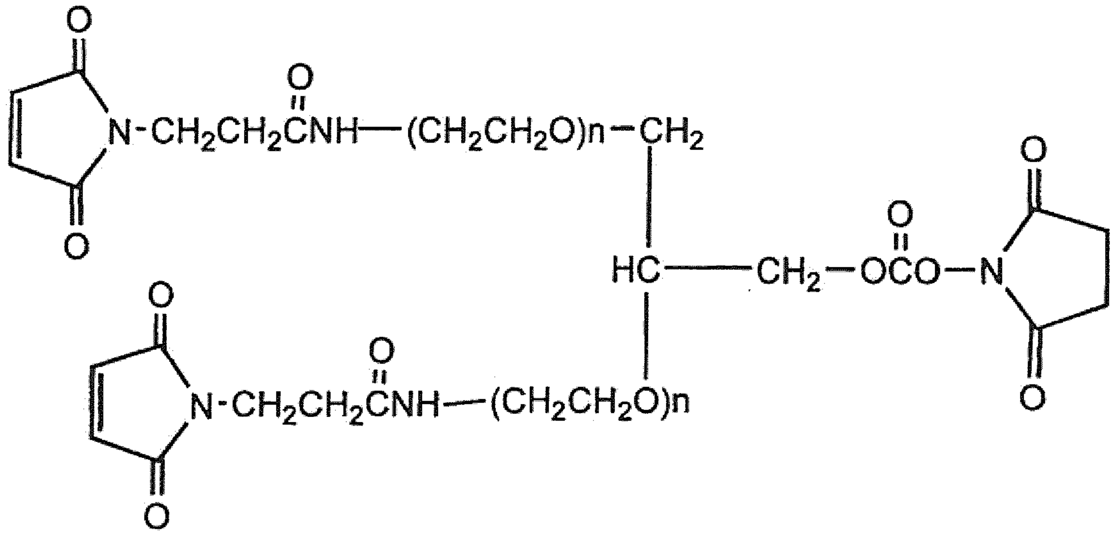

(a12) n = about 218

(Example 13)

GRGDS (Gly-Arg-Gly-Asp-Ser, molecular weight: 490.5) was dissolved in an amount of 2.5 mg (5.1 μmol) in 1 ml of 10 mM phosphoric acid buffer (pH=6.4) . To 200 μl of the solution was added 10 mg (0.5 μmol) of the compound (a12), and reaction was carried out at room temperature for 1 hour, thereby GRGDS being modified at one active carbonate group in the compound. Subsequently, 20 mg (7.4 μmol) of Humanin (Met-Ala-Pro-Arg-Gly-Phe-Ser-Cys-Leu-Leu-Leu-Leu-Thr-Ser-Glu-Ile-Asp-Leu-Pro-Val-Lys-Arg-Arg-Ala, molecular weight: 2, 687.2) was added to the solution, and reaction was carried out at room temperature for 10 hours, thereby Humanin being modified at the two maleimide groups in the compound. Then, 200 μl of the reaction solution was charged onto an SP-Sepharose FF (manufactured by Amersham plc) column and equilibration was performed with 20 mM Tris-HCl buffer (pH 8.2). After equilibration, the buffer to which NaCl had been added so as to be IN was passed through the column to obtain a fraction of a PEG compound modified with one GRGDS and two Humanin. After 20 μl of the fraction and 20 μl of a Tris SDS sample treating liquid were mixed, the mixture was heated in a boiled water bath for 2 minutes and 30 seconds and 20 μl of the solution was analyzed on sodium dodecyl sulfate-polyacrylamide gel electrophoresis. Staining was performed by CBB staining. As a result, through comparison with respective standard samples, it was shown that a compound where the compound (a12) was modified with one GRGDS and two Humanin.

## Claims

**1.** A branched hetero polyethylene glycol represented by the formula [3]:

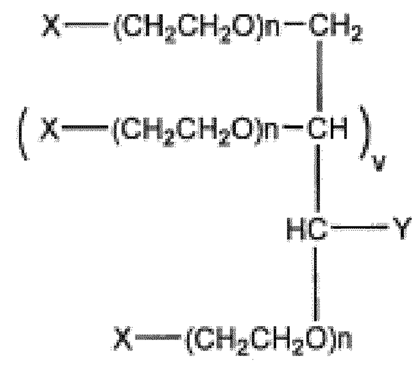

...[3]

,

wherein Y is represented by the formula [6] shown below:

$$Y- = Y' - W'- \quad \ldots \quad [6],$$

wherein the functional group of the atomic group X and the functional group of the atomic group Y' are each selected from the group consisting of an acetal group, an aldehyde group, a maleimide group, a vinylsulfone group, an iodoacetamide group, an active ester group, an active carbonate group, a carboxyl group, an amino group, an aminoxy group, a thiol group, an allyl group, a vinyl group, an acetylene group, and an azide group;
wherein W' is a linker moiety comprising a covalent bond such as an ester bond, a urethane bond, an amide bond, an ether bond, a carbonate bond, a saturated hydrocarbon group containing a secondary amino group, a single bond, or a saturated hydrocarbon group;
n is a number of average added moles for the polyethylene glycol chain and $20 \leq n \leq 2000$; and
wherein v=0.

2. The branched hetero polyethylene glycol according to claim 1, wherein at least one of the functional group of the atomic group X and the functional group of the atomic group Y' is selected from the group consisting of a carboxyl group, an amino group, an aminoxy group, and a thiol group.

3. The branched hetero polyethylene glycol according to any one of claims 1 to 2, wherein (i) n is $120 \leq n \leq 500$ and/or (ii) wherein the group W' is selected from the group consisting of a methylene group, an ethylene group, a trimethylene group, a propylene group, an isopropylene group, a tetramethylene group, a butylene group, an isobutylene group, a pentamethylene group, or a hexamethylene group.

4. An intermediate, which is an intermediate of the branched hetero polyethylene glycol according to any one of claims 1 to 3 and wherein at least one of the functional group of the atomic group X and the functional group of the atomic group Y' is protected by a protecting group.

5. A polyethylene glycol bonded substance, wherein a bio-functional molecule is bonded to an end of the branched hetero polyethylene glycol according to any one of claims 1 to 3.

6. A polyethylene glycol bonded substance, wherein two different bio-functional molecules are bonded to the plural functional groups of the branched hetero polyethylene glycol according to any one of claims 1 to 3.

7. The polyethylene glycol bonded substance according to any one of claims 5 or 6, wherein the bio-functional molecule(s) is/are selected from a protein drug, a polypeptide, an enzyme, an antibody, an antibody medicament, a gene, a nucleic acid compound including an oligonucleotide, a nucleic acid medicament, an anticancer agent, a low-molecular-weight drug, a liposome, a polymer micelle.

8. The polyethylene glycol bonded substance according to any one of claims 5 to 7, wherein the bio-functional molecule for the atomic group X has a molecular weight of 2,000 Da or more, preferably 3,000 Da or more.

9. The polyethylene glycol bonded substance according to any one of claims 5 to 7, wherein the bio-functional molecule

for the atomic group Y' has a molecular weight of 3,000 Da or less, preferably 2,000 Da or less.

**Patentansprüche**

1. Verzweigtes Heteropolyethylenglykol, das durch die Formel [3] dargestellt ist:

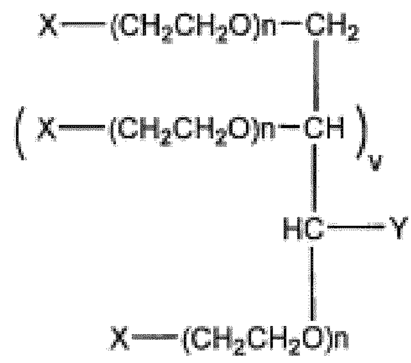

...[3]

worin Y durch die nachstehend gezeigte Formel [6] dargestellt ist:

$$Y- = Y' - W'- \; ... \; [6],$$

worin die funktionelle Gruppe der atomaren Gruppe X und die funktionelle Gruppe der atomaren Gruppe Y' jeweils ausgewählt sind aus der Gruppe bestehend aus einer Acetalgruppe, einer Aldehydgruppe, einer Maleimidgruppe, einer Vinylsulfongruppe, einer Iodacetamidgruppe, einer Aktivestergruppe, einer Aktivcarbonatgruppe, einer Carboxylgruppe, einer Aminogruppe, einer Aminoxygruppe, einer Thiolgruppe, einer Allylgruppe, einer Vinylgruppe, einer Acetylengruppe und einer Azidgruppe;
worin W' eine Verbindungseinheit ist, umfassend eine kovalente Bindung wie eine Esterbindung, eine Urethanbindung, eine Amidbindung, eine Etherbindung, eine Carbonatbindung, eine gesättigte Kohlenwasserstoffgruppe, die eine sekundäre Aminogruppe enthält, eine Einzelbindung oder eine gesättigte Kohlenwasserstoffgruppe;
n eine Anzahl von durchschnittlich zugesetzten Molen für die Polyethylenglykolkette ist und $20 \leq n \leq 2000$; und worin $v = 0$.

2. Verzweigtes Heteropolyethylenglykol gemäß Anspruch 1, worin mindestens eines aus der funktionellen Gruppe der atomaren Gruppe X und der funktionellen Gruppe der atomaren Gruppe Y' ausgewählt ist aus der Gruppe bestehend aus einer Carboxylgruppe, einer Aminogruppe, einer Aminoxygruppe und einer Thiolgruppe.

3. Verzweigtes Heteropolyethylenglykol gemäß Anspruch 1 oder 2, worin (i) n $120 \leq n \leq 500$ ist und/oder (ii) worin die Gruppe W' ausgewählt ist aus der Gruppe bestehend aus einer Methylengruppe, einer Ethylengruppe, einer Trimethylengruppe, einer Propylengruppe, einer Isopropylengruppe, einer Tetramethylengruppe, einer Butylengruppe, einer Isobutylengruppe, einer Pentamethylengruppe oder einer Hexamethylengruppe.

4. Intermediat, das ein Intermediat des verzweigten Heteropolyethylenglykols gemäß mindestens einem der Ansprüche 1 bis 3 ist und worin mindestens eines aus der funktionellen Gruppe der atomaren Gruppe X und der funktionellen Gruppe der atomaren Gruppe Y' mit einer Schutzgruppe geschützt ist.

5. Polyethylenglykol-gebundene Substanz, worin ein biofunktionelles Molekül an ein Ende des verzweigten Heteropolyethylenglykols gemäß mindestens einem der Ansprüche 1 bis 3 gebunden ist.

6. Polyethylenglykol-gebundene Substanz, worin zwei verschiedene biofunktionelle Moleküle an die Vielzahl von funktionellen Gruppen des verzweigten Heteropolyethylenglykols gemäß mindestens einem der Ansprüche 1 bis 3 gebunden sind.

7. Polyethylenglykol-gebundene Substanz gemäß Anspruch 5 oder 6, worin das/die biofunktionelle(n) Molekül(e) ausgewählt ist/sind aus einem Proteinarzneimittel, einem Polypeptid, einem Enzym, einem Antikörper, einem Antikörpermedikament, einem Gen, einer Nukleinsäureverbindung, die ein Oligonukleotid umfasst, einem Nukleinsäuremedikament, einem Antikrebsmittel, einem Arzneimittel mit niedrigem Molekulargewicht, einem Liposom, einer Polymermizelle.

**8.** Polyethylenglykol-gebundene Substanz gemäß mindestens einem der Ansprüche 5 bis 7, worin das biofunktionelle Molekül für die atomare Gruppe X ein Molekulargewicht von 2.000 Da oder mehr, vorzugsweise 3.000 Da oder mehr aufweist.

**9.** Polyethylenglykol-gebundene Substanz gemäß mindestens einem der Ansprüche 5 bis 7, worin das biofunktionelle Molekül für die atomare Gruppe Y' ein Molekulargewicht von 3.000 Da oder weniger, vorzugsweise 2.000 Da oder weniger aufweist.

## Revendications

**1.** Hétéro-polyéthylène glycol ramifié représenté par la formule [3] :

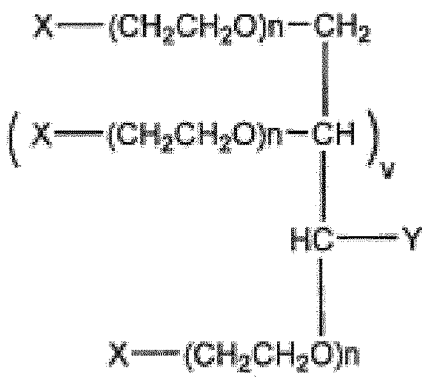

...[3]

dans lequel Y est représenté par la formule [6] ci-dessous :

$$Y- = Y'- W'- \ldots [6],$$

dans lequel le groupe fonctionnel du groupe atomique X et le groupe fonctionnel du groupe atomique Y' sont chacun sélectionnés à partir du groupe constitué par un groupe acétal, un groupe aldéhyde, un groupe maléimide, un groupe vinylsulfone, un groupe iodoacétamide, un groupe ester actif, un groupe carbonate actif, un groupe carboxyle, un groupe amino, un groupe aminoxy, un groupe thiol, un groupe allyle, un groupe vinyle, un groupe acétylène et un groupe azide ;
dans lequel W' est une fraction de liaison comprenant une liaison covalente telle qu'une liaison ester, une liaison uréthane, une liaison amide, une liaison éther, une liaison carbonate, un groupe hydrocarboné saturé contenant un groupe amino secondaire, une liaison simple ou un groupe hydrocarboné saturé ;
n est un nombre moyen de moles ajoutées pour la chaîne de polyéthylène glycol et $20 \leq n \leq 2000$; et
dans lequel v=0.

**2.** Hétéro-polyéthylène glycol ramifié selon la revendication 1, dans lequel au moins un du groupe fonctionnel du groupe atomique X et du groupe fonctionnel du groupe atomique Y' est sélectionné à partir du groupe constitué par un groupe carboxyle, un groupe amino, un groupe aminoxy et un groupe thiol.

**3.** Hétéro-polyéthylène glycol ramifié selon l'une quelconque des revendications 1 à 2, dans lequel (i) n vaut $120 \leq n \leq 500$ et/ou (ii) dans lequel le groupe W' est sélectionné à partir du groupe constitué par un groupe méthylène, un groupe éthylène, un groupe triméthylène, un groupe propylène, un groupe isopropylène, un groupe tétraméthylène, un groupe butylène, un groupe isobutylène, un groupe pentaméthylène ou un groupe hexaméthylène.

**4.** Intermédiaire, qui est un intermédiaire de l'hétéro-polyéthylène glycol ramifié selon l'une quelconque des revendications 1 à 3 et dans lequel au moins l'un du groupe fonctionnel du groupe atomique X et du groupe fonctionnel du

groupe atomique Y' est protégé par un groupe protecteur.

5. Substance liée à du polyéthylène glycol, dans laquelle une molécule biofonctionnelle est liée à une extrémité de l'hétéro-polyéthylène glycol ramifié selon l'une quelconque des revendications 1 à 3.

6. Substance liée à du polyéthylène glycol, dans laquelle deux molécules bio-fonctionnelles différentes sont liées aux multiples groupes fonctionnels de l'hétéro-polyéthylène glycol ramifié selon l'une quelconque des revendications 1 à 3.

7. Substance liée à du polyéthylène glycol selon l'une quelconque des revendications 5 ou 6, dans laquelle la ou les molécules bio-fonctionnelles est/sont sélectionné à partir d'un médicament protéique, d'un polypeptide, d'une enzyme, d'un anticorps, d'un médicament anticancéreux, d'un gène, d'un composé d'acide nucléique incluant un oligonucléotide, d'un médicament à base d'acide nucléique, d'un agent anticancéreux, d'un médicament à faible poids moléculaire, d'un liposome, d'une micelle polymère.

8. Substance liée à du polyéthylène glycol selon l'une quelconque des revendications 5 à 7, dans laquelle la molécule biofonctionnelle pour le groupe atomique X a un poids moléculaire de 2 000 Da ou plus, de préférence de 3 000 Da ou plus.

9. Substance liée à du polyéthylène glycol selon l'une quelconque des revendications 5 à 7, dans laquelle la molécule biofonctionnelle pour le groupe atomique Y' a un poids moléculaire de 3 000 Da ou moins, de préférence de 2 000 Da ou moins.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6362254 B
- WO 03040211 A
- JP 2000001541 B


### Non-patent literature cited in the description


- *Journal of Drug Targeting,* 2007, vol. 15 (10), 672-683
- *BioconjugateChem.,* 2009, vol. 20, 120-128
- *BioconjugateChem.,* 2008, vol. 19, 2156-2162
- *BioconjugateChem.,* 2008, vol. 19, 2321-2331
- *Langmuir,* 2004, vol. 20 (26), 11285-11287